(19) Europäisches Patentamt
European Patent Office
Office européen des brevets

(11) Publication number: 0 048 573
B1

(12) EUROPEAN PATENT SPECIFICATION

(45) Date of publication of patent specification: 01.08.84

(51) Int. Cl.[3]: C 07 C 103/52, A 61 K 37/02

(21) Application number: 81304142.3

(22) Date of filing: 10.09.81

# (54) Polypeptides.

(30) Priority: 19.09.80 US 188804
27.07.81 US 286138

(43) Date of publication of application:
31.03.82 Bulletin 82/13

(45) Publication of the grant of the patent:
01.08.84 Bulletin 84/31

(84) Designated Contracting States:
AT BE CH DE FR IT LI LU NL SE

(56) References cited:
EP - A - 0 025 897
US - A - 4 079 127

CHEMICAL PHARMACEUTICAL BULLETIN, vol. 27, no. 12, 1979 Pharmaceutical Society of Japan, TOKYO (JP) TAKASHI ABIKO et al.: "The effect of a Synthetic Thymosin alpha-1 Fragment on the inhibition of E-Rosette formation by the serum of a patient with nephrotic syndrome" pages 3171-3175

(73) Proprietor: AMERICAN HOME PRODUCTS CORPORATION
685, Third Avenue
New York, New York 10017 (US)

(72) Inventor: McGregor, William Herbert
497, Leboutillier Road
Malvern Pennsylvania 19355 (US)

(74) Representative: Wileman, David Francis et al,
c/o John Wyeth and Brother Limited
Huntercombe Lane South
Taplow Maidenhead Berkshire SL6 OPH (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).



Courier Press, Leamington Spa, England.

**Description**

This invention relates to polypeptides, more particularly to short peptide sequences possessing pharmacological activity, to processes for their preparation, to intermediates used in such processes and to pharmaceutical compositions containing them.

Recent research has established the involvement of the thymus in the functioning of the immune system in mammalian species. It is in the thymus that haemopoietic stem cells become differentiated to mature immunocompetent lymphocytes called T-cells, which circulate to the blood, lymph, spleen and lymph nodes. The T-cells have immunological specificity and are involved in the cell-mediated immune responses, such as graft responses, response to viral infections, response to neoplasms and so forth. The body's response to antigenic material, such as for example in response to bacterial attack, is the province of antibody secreting cells, called B-cells, which are derived from bone marrow stem cells, but which are not differentiated in the thymus. The antibody response to an antigen, in many cases, requires the presence of appropriate T-cells, so that T-cells, and consequently the thymus, are necessary for the body's immune system to make not only cellular immunity responses, but also humoral antibody response. The thymic induction of the necessary differentiation of stem cells to T-cells is mediated by secretions of thymic hormones by the epithelial cells of the thymus.

The great interest in thymic substances, which may be implicated in various aspects of the immune response, has been instrumental in creating a very productive research effort. As a result of this research, a number of thymic substances have been reported in the literature. In an article by Low et al. in The Journal of Biological Chemistry, volume 254, pages 981—993, 1979, there are disclosures regarding the potent immunopotentiating effect of the partially purified extract from thymus tissue termed thymosin Fraction 5. It has been shown that this thymosin fraction corrects some of the deficiencies resulting from lack of thymic function in a number of animal models, as well as in humans with primary immunodeficiency diseases and in immunosuppressed cancer patients.

Further, in the Low et al. article, there is disclosed that thymosin Fraction 5 is composed of several polypeptide components, two of which are designated thymosin $\alpha_1$ and polypeptide $\beta_1$. Of these two major components, thymosin $\alpha_1$ has been demonstrated to be a potent immunologically active thymic polypeptide, which is 10—1,000 times as active as the parent thymosin Fraction 5 when assayed in several biological models, including an *in vivo* mouse mitogen assay, an *in vitro* lymphokine assay measuring production of macrophage inhibitor factor (MIF), an *in vitro* induction of Lyt surface markers on putative T-cell precursors, and an *in vitro* human E-rosette assay measuring the production of T-cells.

The Low et al. article further establishes the sequence of the 28 amino acids comprising thymosin $\alpha_1$.

The present invention relates to short peptide sequences which have been found to exhibit characteristics of the long chain polypeptide isolated and named thymosin $\alpha_1$.

In accordance with this invention, there is provided a group of polypeptides having the structural formula:

$$R_1\text{-Lys-Lys-X-}R_2 \qquad (I)$$

wherein $R_1$ is hydrogen or p-Glu, X is Glu or Asp and $R_2$ is $NH_2$, Val-$NH_2$ or Val-OH, and the pharmaceutically acceptable salts thereof.

In the depicted formula and throughout the specification and claims, where the chirality of an amino acid is not indicated or otherwise stated, it is understood to be of the *L*-series.

The pharmaceutically acceptable salts of the compounds of the invention are those non-toxic addition salts produced by known methods from acids conventionally employed with pharmaceuticals such as hydrochloric, hydrobromic, sulfuric, phosphoric, polyphosphoric, maleic, acetic, citric, benzoic, succinic, malonic and ascorbic.

The polypeptides of the invention are able to directly induce the maturation of T-cell populations, thereby affecting the very early stages of T-cell development. These effects are observable with concentrations as low as 1—100 ng/ml., making these compounds useful in the therapeutical treatment of a number of disorders of the immune response. Because the compounds perform certain of the thymic functions, they have application in various thymic function and immunity areas. Thus, the compounds can help to restore immune function and augment specific lymphocyte functions in children with hypothymic function and in adults with a variety of T-cell disorders, including cancer and autoimmune diseases. The polypeptides will increase or assist in therapeutic stimulation of cellular immunity and thereby become useful in the treatment of diseases involving chronic infection *in vivo*, such as fungal or mycoplasma infection, tuberculosis, leprosy, acute and chronic viral infections. Further, the compounds are useful in areas involving immunity deficiencies such as DiGeorge Syndrome, and in treating immunosuppressed cancer patients. The compounds may also be of therapeutic value in certain autoimmune diseases, such as systemic lupus erythematosus.

An important characteristic of the polypeptides is their *in vivo* ability to restore cells with the characteristic of the T-cells. The peptides are highly active in very low concentrations ranging from 1

nanogram per ml. up to 100 nanograms per ml. The carrier for the compounds may be any of the well known carriers for this purpose including normal saline solutions, preferably with a protein diluent such as bovine serum albumin to prevent adsorptive losses to glassware at these low concentrations.

Accordingly this invention also provides a pharmaceutical composition comprising a compound of formula I or a pharmaceutically acceptable salt thereof and a pharmaceutically acceptable carrier.

The compounds of formula I may be prepared by a process of this invention from intermediates having the formula:

$$\begin{array}{c} \;\;A\;\;\;\;A_1\;B \\ \;\;|\;\;\;\;\;|\;\;\;| \\ R_3\text{-Lys-Lys-X-(Val)}_n\text{-}R_4 \end{array} \qquad (II)$$

or a salt thereof, wherein X is Glu or Asp, n is 0 or 1, $R_3$ is hydrogen, p-Glu or an $\alpha$-amino protecting group; $R_4$ is —$NH_2$, a benzhydrylamine polystyrene resin support, or when n is 1, $R_4$ also represents $OR_5$ where $R_5$ is hydrogen or a carboxy protecting group or $CH_2$ [polystyrene resin support]; A and $A_1$ are each independently hydrogen or protecting groups for the $\varepsilon$-amino group of lysine; and B is hydrogen or a protecting group for the carboxy group of glutamic or aspartic acid, with the proviso that when $R_4$ is amino or OH at least one of $R_3$, A, $A_1$ and B is a protecting group; some of which compounds are known from Chem. Pharm. Bull. 27, 3171 (1979). Included in this invention are novel intermediates of formula II in which $R_3$, $R_4$, A, $A_1$, B and n are as defined above but with the further proviso that when $R_3$, A, $A_1$ and B are protecting groups, n is 1 and X is Glu then $R_4$ is $NH_2$ or a benzhydrylamine resin support.

Examples of $R_3$ when an $\alpha$-amino protecting group are (1) acyl type protecting groups illustrated by the following: formyl, trifluoroacetyl, phthalyl, *p*-toluenesulfonyl (tosyl) and o-nitrophenylsulfenyl; (2) aromatic urethane type protecting groups illustrated by benzyloxycarbonyl and substituted benzyloxycarbonyl such as *p*-chlorobenzyloxycarbonyl, *p*-nitrobenzyloxycarbonyl, *p*-bromobenzyloxycarbonyl and *p*-methoxybenzyloxycarbonyl; (3) aliphatic urethane protecting groups illustrated by *tert*-butyloxycarbonyl, diisopropylmethoxycarbonyl, isopropyloxycarbonyl, allyloxycarbonyl, 2,2,3-trichloroethoxycarbonyl and amyloxycarbonyl; (4) cycloalkyl urethane type protecting groups illustrated by cyclopentyloxycarbonyl; adamantyloxycarbonyl, cyclohexyloxycarbonyl; (5) thiourethane type protecting groups such as phenylthiocarbonyl; (6) alkyl type protecting groups as illustrated by triphenylmethyl (trityl); (7) trialkylsilane groups such as trimethylsilane. The preferred $\alpha$-amino protecting group is *tert*-butyloxycarbonyl.

Examples of protecting groups for the side chain $\varepsilon$-amino group of lysine are independently tosyl, amyloxycarbonyl, t-butyloxycarbonyl diisopropyloxycarbonyl, benzyloxycarbonyl, halobenzyloxycarbonyl and p-nitrobenzyloxycarbonyl. Preferably A and $A_1$ represent 2-chlorobenzyloxycarbonyl.

Examples of protecting groups for the carboxy of glutamic and aspartic acid and for $R_5$, are groups which form esters, e.g. benzyl, methyl, ethyl, t-butyl.

This invention also provides processes for preparing the compounds of the invention.

The compounds of formula I may be prepared by removing any protecting groups and the resin support when present from a peptide of formula II as defined above, if desired converting a compound of formula I obtained to a pharmaceutically acceptable salt.

Removal of the protecting groups may be effected by methods known in the art for the respective protecting groups. Preferably the protecting groups and the resin support when present are removed in a single step, for example, by using hydrogen fluoride, preferably in the presence of anisole.

Protecting groups and methods for removing them are well known in the art—see for example E. Schroder and K. Lubke "The Peptides" volume 1 Academic Press, New York and London, 1965.

The resin support when present may be cleaved at the same time as removal of the protecting groups, e.g. by using hydrogen fluoride, preferably in the presence of anisole. Alternatively the resin may be cleaved prior to deprotection.

When $R_4$ represents a benzhydrylamine resin support cleaving by HF produces a peptide having a C-terminal amide group, i.e. $R_4$ is $NH_2$. When $R_4$ represents a —$OCH_2$ [polystyrene resin support] cleaving by HF produces a compound of formula I where $R_4$ is OH. Cleaving of the latter resin supported peptide by transesterification, e.g. using dimethylaminoethanol followed by mild hydrolysis in the manner described by Barton et al. JACS, 95, 4501-6 1973, produces a peptide of formula II having a terminal carboxyl group (i.e. $R_4$ is OH).

The compounds of this invention may be built up by classical or solid phase synthesis.

In the classical method as applied to the compounds of formula II the desired peptide sequence is built up by condensing amino acids or their derivatives or groups of amino acids which are protected if necessary. The C-terminal amino acid, either Glu, Asp or Val when necessary is in the form of an amide with the carboxy of Glu and Asp being protected. The condensation reactions may be carried out using methods generally known to form amide bonds in peptide and penicillin chemistry. To promote facile condensation of the amino acids it is preferred to employ a condensing agent. Examples of condensing agents are carbodiimides; e.g. N,N'-dicyclohexylcarbodiimide, (DCC), N,N'-diisopropylcarbodiimide. Alternatively the condensation may be effected by activating one or both of the terminal groups. Examples of the activated form of the terminal carboxyl are the acid chloride, anhydride, azide and the

activated ester. The condensation reactions should be compatible with the protecting group(s) on the amino acids. Methods of activating amino acids prior to coupling and coupling methods themselves are well known in the art—see for example the textbook of Schroder and Lubke mentioned above.

Compounds of formula II as hereinbefore defined wherein $R_4$ is $—NH_2$ or $OR_5$ where $R_5$ is hydrogen or a carboxy protecting group may be prepared by a process which comprises coupling the requisite suitably protected and/or activated amino acids or groups of amino acids in which the precursor C-terminal amino acid (either Glu, Asp or Val) is in the form of an amide when necessary, to give the desired sequence, if desired removing one or some of the protecting groups. Such a process is within the scope of this invention when applied to preparation of the novel intermediates of this invention having formula II.

Typical solid phase procedures utilize either a benzhydrylamine or chloromethylated polystyrene resin.

In the well known solid phase method, e.g. as described by Stewart et al., Solid Phase Peptide Synthesis, Freeman and Co., San Francisco, 1969, as applied to compounds of this invention, $\alpha$-amino protected valine, glutamic acid or aspartic acid (the latter two having protected carboxy side chains) are attached to a benzhydrylamine polystyrene resin or valine is attached to a chloro- or hydroxymethylated polystyrene resin followed by removal of the $\alpha$-amino protecting group with trifluoroacetic acid in methylene chloride, trifluoroacetic acid alone or HCl in dioxane. The deprotection is carried out at temperatures between about 0°C and room temperature. Other standard cleaving reagents and conditions for removal of specific $\alpha$-amino protecting groups may be used as described in Schroder and Lubke, "The Peptides", *1*, 72—75 (Academic Press, 1965). After removal of the $\alpha$-amino protecting group the subsequent protected amino acids are coupled individually to the resin supported sequence, seriatim. Alternatively, small peptide fragments may be prepared by the solution method and introduced into the solid phase reactor in about a four fold excess. The coupling is preferably carried out in dimethylformamide, methylene chloride, or a mixture of the two solvents. The success of each coupling reaction at each stage of the synthesis is determined by the ninhydrin reaction as described by E. Kaiser et al., Analyt. Biochem., *34*, 595 (1970). Where incomplete coupling has occurred, the reaction is repeated before the $\alpha$-amino protecting group is removed for introduction of the next amino acid or amino acid sequence. The coupling reagent preferably is diisopropylcarbodiimide.

Compound of formula II wherein $R_4$ represents a benzhydrylamine resin support or $OCH_2$[polystyrene resin support] may be prepared by a process which comprises coupling the requisite amino acids or groups of amino acids suitably protected and/or activated under solid phase synthesis conditions to a benzhydrylamine polystyrene resin, or a hydroxy- or chloro-methylated resin support, if desired, removing one or some of the protecting groups. Such a process is within the scope of this invention when applied to the preparation of the novel intermediates of this invention having formula II.

After the desired amino acid sequence has been synthesized, the polypeptide may be removed from the resin support by treatment with hydrogen fluoride and anisole to obtain the fully deprotected polypeptide. The polypeptide can be purified by one or more purification techniques, including gel filtration, high pressure preparative liquid chromatography and partition chromatography.

The ultimate fully protected, resin bound polypeptides specifically exemplified are *L* - (5 - oxoprolyl) - $N^{\varepsilon}$ - 2 - chlorobenzyloxycarbonyl - *L* - lysyl - $N^{\varepsilon}$ - 2 - chlorobenzyloxycarbonyl - *L* - lysyl - O - benzyl - *L* - glutamyl - L - valyl benzhydrylamine polystyrene amide; *L* - (5 - oxoprolyl) - $N^{\varepsilon}$ - 2 - chlorobenzyloxycarbonyl - *L* - lysyl - $N^{\varepsilon}$ - 2 - chlorobenzyloxycarbonyl - L - lysyl - O - benzyl - *L* - glutamyl benzhydrylamine polystryene amide; and $N^{\alpha}$ - t - Boc - $N^{\varepsilon}$ - 2 - chlorobenzyloxycarbonyl - *L* - lysyl - $N^{\varepsilon}$ - 2 - chlorobenzyloxycarbonyl - *L* - lysyl - O - benzyl - *L* - glutamyl - *L* - valyl benzhydrylamine polystyrene amide; $N^{\alpha}$ - t - Boc - $N^{\varepsilon}$ - 2 - chlorobenzyloxycarbonyl - *L* - lysyl - $N^{\varepsilon}$ - 2 - chlorobenzyloxycarbonyl - *L* - lysyl - O - benzyl - *L* - glutamyl - *L* - valyl hydroxymethyl polystyrene ester and $N^{\alpha}$ - t - Boc - $N^{\varepsilon}$ - 2 - chlorobenzyloxycarbonyl - *L* - lysyl - $N^{\varepsilon}$ - 2 - chlorobenzyloxycarbonyl - *L* - lysyl - O - benzyl - *L* - aspartyl - *L* - valyl - benzyhydrylamine polystyrene resin.

The protecting groups employed throughout the solid phase synthesis are well known to the art. The $\alpha$-amino protecting groups employed with each amino acid introduced in sequence of the ultimate polypeptide are of the (1) acyl type protecting groups illustrated by the following: formyl, trifluoroacetyl, phthalyl, *p*-toluenesulphonyl (tosyl), o-nitrophenylsulfenyl, etc.; (2) aromatic urethane type protecting groups illustrated by benzyloxycarbonyl and substituted benzyloxycarbonyl such as *p*-chlorobenzyloxycarbonyl, *p*-nitrobenzyloxycarbonyl, *p*-bromobenzyloxycarbonyl, *p*-methoxybenzyloxycarbonyl; (3) aliphatic urethane protecting groups illustrated by tert-butyloxycarbonyl, diisopropylmethoxycarbonyl, isopropyloxycarbonyl, allyloxycarbonyl, 2,2,3-trichloroethoxycarbonyl, amyloxycarbonyl; (4) cycloalkyl urethane type protecting groups illustrated by cyclopentyloxycarbonyl, adamantyloxycarbonyl, cyclohexyloxycarbonyl; (5) thio urethane type protecting groups such as phenylthiocarbonyl; (6) alkyl type protecting groups as illustrated by triphenylmethyl (trityl); (7) trialkylsilane groups such as trimethylsilyl. The preferred $\alpha$-amino protecting group is tert-butyloxycarbonyl.

Protection for the carboxy group of aspartic and glutamic acid is any ester or anhydride which is not removed during removal to the $\alpha$-amino protecting groups. Preferably the benzyl ester is employed to protect the carboxy group.

Protection for the side chain amino group of amino acids such as lysine, may be, for example, by tosyl, *t*-amyloxycarbonyl, *t*-butyloxycarbonyl, diisopropylmethoxycarbonyl, benzyloxycarbonyl, halobenzyloxycarbonyl and p-nitrobenzyloxycarbonyl; the 2-chlorobenzyloxycarbonyl group being preferred.

In selecting a particular side-chain protecting group to be used in the classical or solid phase synthesis of the peptides of this invention, the following rules should be followed: (a) the side-chain protecting group must be stable to the reagent and under the reaction conditions selected for removing the $\alpha$-amino protecting group at each step of the synthesis, (b) the protecting group must retain its protecting properties (i.e. not be split off under coupling conditions), and (c) the side-chain protecting group must be removable upon the completion of the synthesis containing the desired amino acid sequence under reaction conditions that will not alter the peptide chain.

The following Examples 1—8 illustrate the preparation of compounds of this invention; Example 9 gives pharmacological test results:

Example 1

*L*-(5-oxoprolyl)-$N^{\varepsilon}$-2-chlorobenzyloxycarbonyl-*L*-lysyl-$N^{\varepsilon}$-2-chlorobenzyloxycarbonyl-*L*-lysyl-O-benzyl-*L*-glutamyl-*L*-valyl benzhydrylamine polystyrene amide

In a solid phase peptide synthesizer, 6 g of benzhydrylamine hydrochloride resin (Bachem) is neutralized twice with 30% v/v triethylamine in methylene chloride for five minutes each and washed successively with methylene chloride ($MeCl_2$) 1 time and twice with dimethylformamide (DMF) and coupled with 3 g *t*-Boc-*L*-valine, 2 g hydroxybenzotriazole and 2 ml dissopropylcarbodiimide in DMF overnight. After successive washings with DMF (1 time), $MeCl_2$ (2 times), MeOH (1 time) and $MeCl_2$ (3 times) the resin gives a slightly positive ninhydrin test and is washed with 30% v/v triethylamine in DMF (1 time) and DMF (2 times). The resin is recoupled with 3 g *t*-Boc-*L*-valine, 2 g hydroxybenzotriazole and 2 ml dissopropylcarbodiimide in DMF overnight.

After successive washings as before [DMF (1 time), $MeCl_2$ (2 times), MeOH (1 time) and $MeCl_2$ (2 times)] the resin gives a trace positive ninhydrin test and is deprotected for 30 minutes with 50% v/v trifluoroacetic acid in $MeCl_2$ followed by successive washing with $MeCl_2$ (1 time), 30% v/v triethylamine in DMF (2 times) and DMF (2 times). The valyl-resin is coupled with 6.7 g *t*-Boc-$\gamma$-benzyl-L-glutamic acid, 3 g hydroxybenzotriazole and 3 ml diisopropylcarbodiimide in DMF overnight.

After successive washing, as previously, with DMF, $MeCl_2$, MeOH and $MeCl_2$ the resin gives a slight positive ninhydrin test, is further washed with 30% v/v triethylamine in DMF (1 time) followed by DMF (2 times) and recoupled with 3.3 g *t*-Boc-$\gamma$-benzyl-*L*-glutamic acid, 1.5 g hydroxybenzotriazole and 3 ml diisopropylcarbodiimide in DMF overnight. After the usual washings the resin gives a trace ninhydrin positive reaction and the terminal amino function is deprotected with trifluoroacetic acid for 30 minutes. The resin is neutralized and washed as described in the previous deprotection step. The dipeptidyl resin is coupled with 5 g *t*-Boc-$\varepsilon$-2-Cl-carbobenzoxy-*L*-lysine, 2 g hydroxybenzotriazole and 2 ml diisopropylcarbodiimide in DMF overnight.

The peptidyl resin, being ninhydrin negative, is deprotected with trifluoroacetic acid, neutralized and washed as in the previous deprotections and coupled with 5 g of *t*-Boc-$\varepsilon$-2-Cl-carbobenzoxy-L-lysine, 2 g hydroxybenzotriazole and 2 ml diisopropylcarbodiimide in DMF over a weekend. After the usual washings, the peptidyl resin is still ninhydrin positive and is recoupled with 5 g *t*-Boc-$\varepsilon$-2-Cl-carbobenzoxy-*L*-lysine, 2 g hydroxybenzotriazole and 2 ml diisopropylcarbodiimide in DMF overnight. After the usual washings the peptidyl resin is still slightly ninhydrin positive and is further coupled with 10 g *t*-Boc-2Cl-carbobenzoxy-*L*-lysine, 4 g hydroxybenzotriazole and 4 ml diisopropylcarbodiimide overnight in DMF. After washing in the usual manner the peptidyl-resin is trace ninhydrin positive, and the terminal amino group is deprotected with trifluoroacetic acid. The resin is neutralized and washed as previously and coupled with 6 g pyro-*L*-glutamic acid, 4 g hydroxybenzotriazole and 4 ml diisopropylcarbodiimide in DMF overnight. The peptidyl resin after the usual washings is still slightly ninhydrin positive and so is recoupled with 6 g pyro-*L*-glutamic acid, 4 g hydroxybenzotriazole and 4 ml diisopropylcarbodiimide in DMF overnight. A second recoupling with the same reagents is necessary to obtain a peptidyl-resin which is trace ninhydrin positive. After washing with ethyl ether and drying the weight is 8 g.

Example 2

*L*-(5-oxoprolyl)-*L*-lysyl-*L*-lysyl-*L*-$\alpha$-glutamyl-*L*-valinamide

8 Grams of the peptidyl-resin of the previous example is cleaved and deprotected with HF in the presence of 8 ml of anisole for 1 hour at 0°C. The HF is removed *in vacuo* and the residue washed 3 times with ethyl ether, dried in a current of nitrogen and triturated with 150 ml of 0.2N HOAc for five minutes and filtered. The filtrate is lyophilized giving 222 mg of crude p-Glu-Lys-Lys-Glu-Val-$NH_2$ . 2 HOAc.

150 mg of crude peptide is chromatographed on Sephadex G-10 using 0.2 *N*-acetic acid as solvent, and 1 ml fractions are collected at a flow rate of 15 ml per hour. Sephadex is a Registered Trade Mark. Collected fractions 53—63 are combined on the basis of TLC silica gel (Merck), BAW,

4:1:5 system (n-butanol:acetic acid:water) using ninhydrin and peptide-chlorine spray for detection ($R_f$ 0.06) and lyophilized to yield 67 mg of the title compound as the diacetate salt.

Amino acid analysis of the product gave the following: Glu 2.2, Lys 2.1, Val 1.0, $NH_3$·1.1.

Example 3

*L*-(5-oxoprolyl)-$N^{\varepsilon}$-2-chlorobenzyloxycarbonyl-*L*-lysyl-$N^{\varepsilon}$-2-chlorobenzyloxycarbonyl-*L*-lysyl-O-benzyl-*L*-glutamylbenzhydrylamine polystyrene amide

In a solid phase peptide synthesizer, 7 g of benzhydrylamine hydrochloride resin (Bachem) is neutralized twice for five minutes each with 30% v/v triethylamine in methylene chloride and washed successively with methylene chloride (1 time) and DMF (2 times followed by coupling with 10 gm *t*-Boc-$\gamma$-benzyl-*L*-glutamic acid, 4 g hydroxybenzotriazole and 4 ml diisopropylcarbodiimide in DMF overnight. The resin after successive washings with DMF (1 time), $MeCl_2$ (2 times), MeOH (1 time) and $MeCl_2$ (2 times) is slightly ninhydrin positive and is recoupled with 10 g *t*-Boc-$\gamma$-benzyl-*L*-glutamic acid, 4 g hydroxybenzotriazole and 4 ml diisopropylcarbodiimide in DMF overnight.

After successive washings as before, the resin gives a positive trace ninhydrin test and is deprotected with 50% v/v trifluoroacetic acid in MeCl (containing 0.5% w/v dithioerythritol (DTE)) for 30 minutes followed by washing once with $MeCl_2$, 30% v/v triethylamine in DMF (2 times) and DMF (2 times). The glutamyl-resin is coupled with 10 g *t*-Boc-$\varepsilon$-2-Cl carbobenzyloxy-L-lysine, 4 g hydroxybenzotriazole and 4 ml diisopropylcarbodiimide in DMF overnight. After washing successively with DMF, $MeCl_2$, MeOH and $MeCl_2$ as before, the resin is ninhydrin trace positive and is deprotected with trifluoroacetic acid as before. After washing and neutralizing as previously described for this step, the dipeptidyl resin is coupled with 10 grams t-Boc-$\varepsilon$-2-Cl-carbobenzoxy-L-lysine, 4 grams hydroxybenzotriazole and 4 ml diisopropylcarbodiimide in DMF. After washing as usual, the resin is ninhydrin trace positive, is deprotected with trifluoroacetic acid, washed, neutralized with triethylamine and washed as previously described at this stage, then coupled with 6 g pyro-*L*-glutamic acid, 4 g hydroxybenzotriazole and 4 ml diisopropylcarbodiimide in DMF overnight. After washing, the resin is still slightly ninhydrin positive and is recoupled with 6.5 g pyroglutamic acid, 4 g hydroxybenzotriazole and 4 ml diisopropylcarbodiimide in DMF as usual. After the usual washing at this stage a slightly ninhydrin positive test is obtained for the peptidyl-resin and it is washed once with ether and dried *in vacuo* for cleavage.

Example 4

*L*-(5-oxoprolyl)-*L*-lysyl-*L*-lysyl-*L*-glutamamide

The protected peptidyl resin of Example 3 is deprotected and cleaved with HF in the presence of 8 ml of anisole, for 1 hour at 0°C and the HF removed *in vacuo* overnight. The residue is washed three times with ethyl ether, dried in a current of nitrogen and triturated with 150 ml of 0.2 *N* HOAc for five minutes and filtered. The filtrate is lyophilized giving 1.65 g of crude pGlu-Lys-Lys-Glu-$NH_2$ . 2 HOAc.

150 mg of crude peptide acetate is purified on Sephadex G-10 using 0.2 *N* HOAc as solvent, and 1 ml fractions are collected at a flow rate of 15 ml per hour. Tubes 59—63 were combined on the basis of TLC silica gel (Merck), nBuOH, HOAc, $H_2O$ 4:1:5 system using ninhydrin and peptide-chlorine spray for detection ($R_f$ 0.0) and lyophilized to yield 63 mg of the title compound as the diacetate salt.

Amino acid analysis of the product gave the following: Lys, 0.97, Glu 1.0.

Example 5

$N^{\alpha}$-t-Boc-$N^{\varepsilon}$-2-chlorobenzyloxycarbonyl-*L*-lysyl-$N^{\varepsilon}$-2-chlorobenzyloxycarbonyl-*L*-lysyl-O-benzyl-*L*-glutamyl-*L*-valyl benzhydrylamine polystyrene amide

In a solid phase peptide synthesizer, 6 g of benzhydrylamine resin (Bachem) is neutralized twice with 30% v/v triethylamine in $MeCl_2$ for 5 minutes each and washed once with $MeCl_2$ and twice with DMF followed by coupling with 6 g t-Boc-*L*-valine, 4 g hydroxybenzotriazole and 4 ml diisopropylcarbodiimide in DMF for 15 hours. After washing with DMF (1 time), $MeCl_2$ (2 times), MeOH (1 time) and $MeCl_2$ (3 times) the resin is slightly ninhydrin positive and is recoupled with 6 g t-Boc-*L*-valine, 4 g hydroxybenzotriazole and 4 ml diisopropylcarbodiimide in DMF for 15 hours. The washing procedure is repeated and the resin again found to be slightly ninhydrin positive. It is recoupled a second time with 6 g t-Boc-*L*-valine, 4 g hydroxybenzotriazole and 4 ml diisopropylcarbodiimide in DMF for 15 hours. After the previously described washing, the resin is ninhydrin negative, is deprotected with trifluoroacetic acid for 30 minutes followed by washing once with $MeCl_2$, twice with 30% v/v triethylamine in DMF and twice with DMF. It is coupled with 10 g t-Boc-$\gamma$-benzyl-*L*-glutamic acid, 4 g hydroxybenzotriazole and 4 ml diisopropylcarbodiimide in DMF over 15 hours. After the previously described washing procedure the peptidyl resin is ninhydrin negative, and is deprotected with trifluoroacetic acid as previously described. After washing with triethylamine in DMF and with DMF as previously described the peptidyl-resin is coupled with 10 g t-Boc-$\varepsilon$-2-Cl-carbobenzoxy-*L*-lysine, 4 g hydroxybenzotriazole and 4 ml diisopropylcarbodiimide in DMF for 15 hours. After the usual washing, the peptidyl-resin is ninhydrin negative and is deprotected with trifluoroacetic acid as described previously, washed in the usual way at this stage and coupled with 10 g t-Boc-$\varepsilon$-2-Cl-carbobenzoxy-*L*-lysine, 4 g hydroxybenzotriazole and 4 ml of diisopropylcarbodiimide in DMF as usual. After washing in the usual fashion, the peptidyl resin is ninhydrin negative, is washed with diethylether and dried *in vacuo*.

Example 6

L-lysyl-L-lysyl-L-glutamyl-L-valineamide

The protected resin of Example 5 is deprotected and cleaved from the resin with HF in the presence of 8 ml of anisole for 1 hour at 0°C, and the excess HF removed *in vacuo*. The residue is washed 3 times with ethyl ether, dried in a current of nitrogen, triturated with 150 ml 0.2*N* acetic acid 10 minutes, filtered and the filtrate lyophilized, giving 719 mg of crude H-Lys-Lys-Glu-Val-$NH_2$ . 3 HOAc.

105 mg of crude peptide is chromatographed on Sephadex G-10 using 0.2*N* acetic acid as solvent and collecting 1 ml fractions at a flow rate of 15 ml per hour. Tubes 52—57 were combined on the basis of TLC silica gel (Merck) BAW (4:1:5) System using ninhydrin detection ($R_f$ 0.0) and lyophilized to yield 54 mg of the title compound as the triacetate salt.

Amino acid analysis of the product gave the following: Glu 1.0, Lys 1.97, Val 0.98.

Example 7

*L*-lysyl-*L*-lysyl-*L*-aspartyl-*L*-valineamide

a) Using an analogous procedure to Example 5 but substituting t-Boc-$\beta$-benzyl-*L*-aspartic acid for t-Boc-$\gamma$-benzyl-*L*-glutamic acid, the following resin supported peptide was prepared: $N^\alpha$-t-Boc-$N^\epsilon$-2-chlorobenzyloxycarbonyl-*L*-lysyl-$N^\epsilon$-2-chlorobenzyloxycarbonyl-*L*-lysyl-O-benzyl-*L*-aspartyl-*L*-valyl benzhydrylamine polystyrene amide.

b) The protected resin of Example 7a was deprotected in the manner described in Example 6 to give the title compound (yield 115 mg) as the diacetate salt. TLC silica gel, BAW (4:1:5) System gave $R_f$=0.05.

Amino acid analysis of the product gave the following: Val 1.0, Lys 2.1, Asp 1.1, $NH_3$ 1.0.

Example 8

*L*-lysyl-*L*-lysyl-*L*-glutamyl-*L*-valine-OH

a) Using a procedure analogous to Example 5 but using a chloromethylated polystyrene resin support instead of a benzhydrylamine resin support the following protected resin was prepared: $N^\alpha$-t-Boc-$N^\epsilon$-2-chlorobenzyloxycarbonyl-*L*-lysyl-N′-2-chlorobenzyloxycarbonyl-*L*-lysyl-O-benzyl-*L*-glutamyl-L-valyl-hydroxymethyl polystyrene ester.

b) The above mentioned protected resin from Example 8a was deprotected in the manner of Example 6 to give the title compound as the diacetate salt (70 mg). TLC silica gel, BAW (4:1:5) System gave $R_f$=0.03.

Amino acid analysis of the product gave the following: Glu 1.0, Val 1.0, Lys 2.0.

Example 9

The activity of the compounds of Examples is determined according to the following procedure:

T lymphocytes are isolated from spleens of male CBA/J or NZB mice. Cell homogenates are prepared in Hank's balanced salt solution (HBSS). After removal of larger particles and repeated washing of the cells in HBSS they are suspended in minimum essential medium (MEM) and passed through a glass wool column to remove macrophages. The cells are then incubated on a nylon wool column at 37°C., 95% air, 5% $CO_2$, for 45 minutes. The non-adherant T lymphocytes are then eluted from the column, counted, and adjusted to $20\times10^6$ cells/ml. 50 $\mu$l of cells are cultured (37°C, 95% air, 5% $CO_2$) with compound, for 48 hours before addition of 0.5 $\mu$Ci of 3H-thymidine for the last 16 hours of culture. The total volume of the culture system is 200 $\mu$l. The cells are then harvested on a multiple automatic sample harvester (Mash II), the glass fiber filter discs placed in 10 ml of xylene base scintillation fluid, and counted for 1 minute in a liquid scintillation counter. Results are reported as CPM$\pm$SE. Levamisole is used as the standard. Comparisons are made between counts obtained with control cultures and cultures containing compound and a determination made as to whether the compounds are active at the dosage tested. The findings are summarized in Table 1.

TABLE 1

| Compound | Concentration (ng/culture) | N* | $^3$H-Thymidine uptake CPM±S.E. | p |
|---|---|---|---|---|
| Control | 0 | 7 | 49,519±4048 | |
| Levamisole | 5 | 8 | 70,353±1561 | <0.05 |
| p-Glu-Lys-Lys-Glu-Val-$NH_2$ | 0 | 10 | 9,876±951 | |
| | 1.5 | 5 | 23,736±3417 | <0.01 |
| | 6.0 | 5 | 31,236±2004 | <0.01 |
| | 25.0 | 5 | 26,026±3537 | <0.01 |
| | 100.0 | 5 | 27,830±1749 | <0.01 |
| H-Lys-Lys-Glu-Val-$NH_2$ | 0 | 7 | 49,519±4048 | |
| | 1.5 | 3 | 72,713±3633 | <0.01 |
| | 6.0 | 4 | 77,979±3760 | <0.05 |
| | 25.0 | 3 | 67,712±6004 | N.S. |
| | 100.0 | 4 | 51,021±5527 | N.S. |
| p-Glu-Lys-Lys-Glu-$NH_2$ | 0 | 7 | 49,519±4048 | |
| | 1.5 | 3 | 73,407±3333 | <0.05 |
| | 6.0 | 3 | 78,727±7102 | <0.05 |
| | 25.0 | 4 | 73,932±6944 | <0.05 |
| | 100.0 | 3 | 67,489±5190 | N.S. |
| H-Lys-Lys-Glu-Val-OH | 0 | 10 | 2,789±148 | |
| | 2.5 | 5 | 3,931±140 | <0.05 |
| | 10.0 | 5 | 3,745±172 | <0.05 |
| | 25.0 | 5 | 4,397±349 | <0.05 |
| | 100.0 | 4 | 4,235±284 | <0.05 |

*N=number of animals tested.

The results show that the peptides of the invention have marked activity in stimulating the proliferation of T-cells at very low concentration levels.

**Claims for the Contracting States: BE, CH, DE, FR, IT, LI, LU, NL, SE**

1. A polypeptide of formula:

$$R_1\text{-Lys-Lys-X-}R_2 \qquad (I)$$

or

$$R_3\text{-}\overset{A}{\overset{|}{\text{Lys}}}\text{-}\overset{A_1}{\overset{|}{\text{Lys}}}\text{-}\overset{B}{\overset{|}{\text{X}}}\text{-(Val)}_n\text{-}R_4 \qquad (II)$$

and pharmaceutically acceptable salts thereof, wherein

X is Glu or Asp;

$R_1$ is hydrogen or p-Glu;

$R_2$ is $NH_2$, Val-$NH_2$ or Val-OH;

$R_3$ is $R_1$ or an $\alpha$-amino protecting group;

n is 0 or 1;

$R_4$ is $NH_2$, a benzhydrylamine polystyrene resin support or when n is 1 $R_4$ also represents $OR_5$ where $R_5$ is hydrogen, a carboxyl protecting group or —$CH_2$[polystyrene resin support];

A and $A_1$ are independently hydrogen or protecting groups for the $\varepsilon$-amino group of lysine; and

B is hydrogen or a protecting group for the carboxy group of glutamic or aspartic acid, with the provisos that (i) when $R_4$ is amino or OH at least one of $R_3$, A, $A_1$ and B is a protecting group, and (ii) when $R_3$, A, $A_1$ and B are protecting groups, n is 1 and X is Glu then $R_4$ is $NH_2$ or a benzhydrylamine resin support.

2. A compound of formula I as claimed in Claim 1 which is one of the following:

p-Glu-Lys-Lsy-Glu-Val-$NH_2$
p-Glu-Lys-Lys-Glu-$NH_2$
H-Lys-Lys-Glu-Val-$NH_2$
H-Lys-Lys-Glu-Val-OH
H-Lys-Lys-Asp-Val-$NH_2$ or

a pharmaceutically acceptable salt thereof.

3. A compound of formula II as claimed in Claim 1 wherein $R_3$ is an $\alpha$-amino protecting group selected from formyl, trifluoroacetyl, phthalyl, tosyl, o-nitrophenylsulfenyl, benzyloxycarbonyl, p-nitrobenzyloxycarbonyl, p-bromobenzyloxycarbonyl, p-methoxybenzyloxycarbonyl, tert-butyloxycarbonyl, diisopropylmethoxycarbonyl, isopropyloxycarbonyl, allyloxycarbonyl, 2,2,3-trichloroethoxycarbonyl, amyloxycarbonyl, cyclopentyloxycarbonyl, adamantyloxycarbonyl cyclohexyloxycarbonyl, phenylthiocarbonyl, triphenylmethyl and trimethylsilyl.

4. A compound of formula II as claimed in Claim 1 or Claim 3 wherein A and $A_1$ are each selected from tosyl, amyloxycarbonyl, t-butoxycarbonyl, diisopropylmethoxycarbonyl, benzyloxycarbonyl, halobenzyloxycarbonyl and p-nitrobenzyloxycarbonyl.

5. A compound of formula II as claimed in any one of Claims 1, 3 and 4 wherein B is benzyl.

6. A compound of formula II as claimed in Claim 1 which is one of the following

L - (5 - Oxoprolyl) - $N^{\varepsilon}$ - 2 - chlorobenzyloxycarbonyl - L - lysyl - $N^{\varepsilon}$ - 2 - chlorobenzyloxycarbonyl - L - lysyl - O - benzyl - L - glutamyl - L - valyl benzhydrylamine polystyrene amide,

L - (5 - Oxoprolyl) - $N^{\varepsilon}$ - 2 - chlorobenzyloxycarbonyl - L - lysyl - $N^{\varepsilon}$ - 2 - chlorobenzyloxycarbonyl - L - lysyl - O - benzyl - L - glutamyl - benzhydrylamine polystyrene amide,

$N^{\alpha}$ - t - Boc - $N^{\varepsilon}$ - 2 - chlorobenzyloxycarbonyl - L - lysyl - $N^{\varepsilon}$ - 2 - chlorobenzyloxycarbonyl - L - lysyl - O - benzyl - L - glutamyl - L - valyl benzhydrylamine polystyrene amide, or

$N^{\alpha}$ - t - Boc - $N^{\varepsilon}$ - 2 - chlorobenzyloxycarbonyl - *L* - lysyl - $N^{\varepsilon}$ - 2 - chlorobenzyloxycarbonyl - *L* - lysyl - O - benzyl - *L* - aspartyl - L - valyl benzhydrylamine polystyrene amide.

7. A process for preparing a compound of formula I as defined in Claim 1 or a pharmaceutically acceptable salt thereof which comprises removing any protecting groups and cleaving the resin support when present from a compound of formula II

$$\begin{array}{cccc} & A & A_1 & B \\ & | & | & | \\ R_3\text{-} & \text{Lys-} & \text{Lys-} & \text{X-(Val)}_n\text{-}R_4 \end{array} \qquad (II)$$

wherein X is Glu or Asp, $R_3$ is hydrogen, p-Glu or an $\alpha$-amino protecting group, n is 0 or 1, $R_4$ is $NH_2$, a benzhydrylamine polystyrene resin support or when n is 1 $R_4$ also represents $OR_5$ where $R_5$ is hydrogen, a carboxyl protecting group or —$CH_2$[polystyrene resin support], and A and $A_1$ are independently hydrogen or protecting groups for the $\varepsilon$-amino group of lysine; and B is hydrogen or a protecting group for the carboxy group of glutamic or aspartic acid, with the proviso that when $R_4$ is amino or OH at least one of $R_3$, A, $A_1$ and B is a protecting group, and if desired converting the compound of formula I obtained to a pharmaceutically acceptable salt.

8. A process for preparing a compound of formula II as claimed in Claim 1 which comprises one of the following:

a) coupling the requisite amino acids or groups of amino acids suitably protected and/or activated under solid phase synthesis conditions to a benzhydrylamine or hydroxy- or chloro-methylated polystyrene resin and if desired removing one or some of the protecting groups, to give a compound of formula II wherein $R_4$ is benzhydrylamine resin support or O—$CH_2$ [polystyrene resin support], or

b) coupling the requisite suitably protected and/or activated amino acids or groups of amino acids, in which the precursor C-terminal amino acid is in the form of an amide when necessary, if desired removing one or some of the protecting groups, to give a compound of formula II wherein $R_4$ is $NH_2$ or $OR_5$ where $R_5$ is hydrogen or a protecting group.

9. A compound of formula I as claimed in Claim 1 for use as an immunomodulating agent.

10. A pharmaceutical composition comprising a compound of formula I as defined in Claim 1 or a pharmaceutically acceptable salt thereof and a pharmaceutically acceptable carrier.

**Claims for the Contracting State: AT**

1. A process for preparing a compound of formula I

$$R_1\text{-Lys-Lys-X-}R_2 \qquad (I)$$

or a pharmaceutically acceptable salt thereof wherein $R_1$ is hydrogen or p-Glu, X is Glu or Asp, $R_2$ is $NH_2$, Val-$NH_2$ or Val-OH, which comprises removing any protecting groups and the resin support when present from a corresponding compound of formula:

$$\begin{array}{cccc} & A & A_1 & B \\ & | & | & | \\ R_3\text{-} & \text{Lys-} & \text{Lys-} & \text{X-(Val)}_n\text{-}R_4 \end{array} \qquad (II)$$

wherein

n is 0 or 1, $R_3$ is $R_1$ or an $\alpha$-amino protecting group;

$R_4$ is $NH_2$, a benzhydrylamine resin support or when n is 1, $R_4$ also represents $OR_5$ where $R_5$ is hydrogen, a carboxy protecting group or —$CH_2$[polystyrene resin support];

A and $A_1$ are independently hydrogen or protecting groups for the $\varepsilon$-amino group of lysine; and

B is hydrogen or a protecting group for the carboxy group of glutamic or aspartic acid, with the proviso that when $R_4$ is amino or OH at least one of $R_3$, A, $A_1$ and B is a protecting group.

2. A process as claimed in Claim 1 wherein $R_3$ is an $\alpha$-amino protecting group selected from formyl, trifluoroacetyl, phthalyl, tosyl, o-nitrophenylsulfenyl, benzyloxycarbonyl, p-nitrobenzyloxycarbonyl, p-bromobenzyloxycarbonyl, p-methoxybenzyloxycarbonyl tert-butyloxycarbonyl, diisopropylmethoxycarbonyl, isopropyloxycarbonyl, allyloxycarbonyl, 2,2,3-trichloroethoxycarbonyl, amyloxycarbonyl, cyclopentyloxycarbonyl, adamantyloxycarbonyl, cyclohexyloxycarbonyl, phenylthiocarbonyl, triphenylmethyl and trimethylsilyl.

3. A process as claimed in Claim 1 or Claim 2 wherein A and $A_1$ are each selected from tosyl, amyloxycarbonyl, t-butyloxycarbonyl, diisopropylmethoxycarbonyl, benzyloxycarbonyl, halobenzyloxycarbonyl and p-nitrobenzyloxycarbonyl.

4. A process as claimed in any one of Claims 1 to 3 wherein B is benzyl.

5. A process as claimed in Claim 1 in which the prepared compound of formula I is one of the following:

p-Glu-Lys-Lys-Glu-Val-$NH_2$
p-Glu-Lys-Lys-Glu-$NH_2$
H-Lys-Lys-Glu-Val-$NH_2$
H-Lys-Lys-Asp-Val-$NH_2$
H-Lys-Lys-Glu-Val-OH

or a pharmaceutically acceptable salt thereof.

6. A process for preparing a compund of formula

```
A   A1 B
|   |  |
R3-Lys-Lys-X-(Val)n-R4        (II)
```

or a salt thereof, wherein n is 0 or 1, $R_3$ is hydrogen, p-Glu or an $\alpha$-amino protecting group; X is Glu of Asp, $R_4$ is —$NH_2$ or a benzhydrylamine polystyrene resin support or when n is 1 $R_4$ also represents $OR_5$ wherein $R_5$ is hydrogen, a carboxy protecting group or —$CH_2$[polystyrene resin support]; A and $A_1$ are each independently hydrogen or protecting groups for the $\varepsilon$-amino group of lysine; and B is hydrogen or a protecting group for the carboxy group of glutamic or aspartic acid, with the provisos (i) that when $R_4$ is amino or OH at least one of $R_3$, A, $A_1$ and B is a protecting group, and (ii) when $R_3$, A, $A_1$ and B are protecting groups n is1 and X is Glu then $R_4$ is $NH_2$ or a benzhydrylamine resin support which comprises

a) coupling the requisite amino acids or groups of amino acids suitably protected and/or activated under solid phase synthesis conditions to a benzhydrylamine or hydroxy- or chloro-methylated polystyrene resin support and if desired removing one or some of the protecting groups, to give a compound of formula II wherein $R_4$ is a benzhydrylamine resin support or $OCH_2$[polystyrene resin support], or

b) coupling the requisite suitably protected and/or activated amino acid or groups of amino acids, in which the precursor C-terminal amino acid is in the form of an amide when necessary, to give a compound of formula II wherein $R_4$ is $NH_2$ or $OR_5$ wherein $R_5$ is hydrogen or a carboxy protecting group.

7. A process as claimed in Claim 6 wherein $R_3$, A, $A_1$ and B are as defined in any one of Claims 2, 3 and 4.

8. A process as claimed in Claim 6 in which the compound of formula II prepared in one of the following:

L - (5 - Oxoprolyl) - $N^{\varepsilon}$ - 2 - chlorobenzyloxycarbonyl - L - lysyl - $N^{\varepsilon}$ - 2 - chlorobenzyloxycarbonyl - L - lysyl - O - benzyl - L - glutamyl - L - valyl benzhydrylamine polystyrene amide,

L - (5 - Oxoprolyl) - $N^{\varepsilon}$ - 2 - chlorobenzyloxycarbonyl - L - lysyl - $N^{\varepsilon}$ - 2 - chlorobenzyloxycarbonyl - L - lysyl - O - benzyl - L - glutamyl benzhydrylamine polystyrene amide,

$N^{\alpha}$ - t - Boc - $N^{\varepsilon}$ - 2 - chlorobenzyloxycarbonyl - L - lysyl - $N^{\varepsilon}$ - 2 - chlorobenzyloxycarbonyl - L - lysyl - O - benzyl - L - glutamyl - L - valyl benzhydrylamine polystyrene amide, or

$N^{\alpha}$ - t - Boc - $N^{\varepsilon}$ - 2 - chlorobenzyloxycarbonyl - *L* - lysyl - $N^{\varepsilon}$ - 2 - chlorobenzyloxycarbonyl - *L* - lysyl - O - benzyl - *L* - aspartyl - *L* - valyl - benzhydrylamine polystyrene amide.

9. A process for preparing a pharmaceutical composition characterised in that a peptide of formula I as defined in Claim 1 or a pharmaceutically acceptable salt thereof is mixed with a pharmaceutical carrier and brought into a form suitable for therapeutic administration.

**Patentansprüche für die Vertragsstaaten: BE, CH, DE, FR, IT, LI, LU, NL, SE**

1. Ein Polypeptid der Formel

$$R_1\text{-Lys-Lys-X-}R_2 \qquad (I)$$

oder

$$\begin{array}{c} \text{A} \quad \text{A}_1 \quad \text{B} \\ | \quad\;\; | \quad\; | \\ R_3\text{-Lys-Lys-X-(Val)}_n\text{-}R_4 \end{array} \qquad (II)$$

und pharmazeutisch annehmbare Salze hievon, worin
X Glu oder Asp ist,
$R_1$ Wasserstoff oder p-Glu bedeutet,
$R_2$ $NH_2$, Val-$NH_2$ oder Val-OH darstellt,
$R_3$ die Bedeutung $R_1$ hat oder eine $\alpha$-Aminoschutzgruppe ist,
n Null oder 1 ist,
$R_4$ $NH_2$ einen Benzhydrylaminopolystyrolharzträger oder, wenn n 1 ist, $R_4$ auch $OR_5$ darstellt, wobei $R_5$ Wasserstoff, eine Carboxyschutzgruppe oder —$CH_2$[Polystyrolharzträger] bedeutet,
A und $A_1$ unabhängig voneinder Wasserstoff oder Schutzgruppen für die $\varepsilon$-Aminogruppe von Lysin sind, und
B Wasserstoff oder eine Schutzgruppe für die Carboxygruppe von Glutamin- oder Asparaginsäure darstellt, mit den Maßgaben, daß, (i) wenn $R_4$ Amino oder OH ist, zumindest eines von $R_3$, A, $A_1$ und B eine Schutzgruppe ist, und (ii) wenn $R_3$, A, $A_1$ und B Schutzgruppen, n 1 und X Glu sind, $R_4$ die Bedeutung $NH_2$ hat oder ein Benzhydrylaminharzträger ist.

2. Eine Verbindung der Formel (I), wie in Anspruch 1 beansprucht, nämlich eine der folgenden:

p-Glu-Lys-Lys-Glu-Val-$NH_2$
p-Glu-Lys-Lys-Glu-$NH_2$
H-Lys-Lys-Glu-$NH_2$
H-Lys-Lys-Glu-Val-OH
H-Lys-Lys-Asp-Val-$NH_2$ oder

ein pharmazeutisch annehmbares Salz hievon.

3. Eine Verbindung der Formel (II), wie in Anspruch 1 beansprucht, worin $R_3$ eine $\alpha$-Aminoschutzgruppe ist ausgewählt unter Formyl, Trifluoracetyl, Phthalyl, Tosyl, o-Nitrophenylsulfenyl, Benzyloxycarbonyl, p-Nitrobenzyloxycarbonyl, p-Bromobenzyloxycarbonyl, p-Methoxybenzyloxycarbonyl, tert.-Butyloxycarbonyl, Diisopropylmethoxycarbonyl, Isopropyloxycarbonyl, Allyloxycarbonyl, 2,2,3-Trichloräthoxycarbonyl, Amyloxycarbonyl, Cyclopentyloxycarbonyl, Adamantyloxycarbonyl, Cyclohexyloxycarbonyl, Phenylthiocarbonyl, Triphenylmethyl und Trimethylsilyl.

4. Eine Verbindung der Formel (II), wie in Anspruch 1 oder 3 beansprucht, worin A und $A_1$ jeweils ausgewählt sind unter Tosyl, Amyloxycarbonyl, tert.-Butyloxycarbonyl, Diisopropylmethoxycarbonyl, Benzyloxycarbonyl, Halogenbenzyloxycarbonyl und p-Nitrobenzyloxycarbonyl.

5. Eine Verbindung der Formel (II), wie in einem der Ansprüche 1, 3 und 4 beansprucht, worin B Benzyl ist.

6. Eine Verbindung der Formel (II), wie in Anspruch 1 beansprucht, nämlich einer der folgenden:

L - (5 - Oxoprolyl) - $N^{\varepsilon}$ - 2 - chlorobenzyloxycarbonyl - L - lysyl - $N^{\varepsilon}$ - 2 - chlorbenzyloxycarbonyl - L - lysyl - O - benzyl - L - glutamyl - L - valyl - benzhydrylamin - polystyrolamid,
L - (5 - Oxoprolyl) - $N^{\varepsilon}$ - 2 - chlorbenzyloxycarbonyl - L - lysyl - $N^{\varepsilon}$ - 2 - chlorbenzyloxycarbonyl - L - lysyl - O - benzyl - L - glutamyl - benzhydrylamin - polystyrolamid,
$N^{\alpha}$ - t - Boc - $N^{\varepsilon}$ - 2 - chlorobenzyloxycarbonyl - L - lysyl - $N^{\varepsilon}$ - 2 - chlorbenzyloxycarbonyl - L - lysyl - O - benzyl - L - glutamyl - L - valyl - benzhydrylamin - polystryolamid oder
$N^{\alpha}$ - t - Boc - $N^{\varepsilon}$ - 2 - chlorbenzyloxycarbonyl - L - lysyl - $N^{\varepsilon}$ - 2 - chlorbenzyloxycarbonyl - L - lysyl - O - benzyl - L - aspartyl - L - valyl - benzhydrylamin - polystyrolamid.

7. Verfahren zum Herstellen einer Verbindung der Formel (I), wie in Anspruch 1 definiert, oder eines pharmazeutisch annehmbaren Salzes hievon, welches das Entfernen jeglicher Schutzgruppen und Abspalten des Harzträgers, wenn vorhanden, von einer Verbindung der Formel (II)

$$\begin{array}{c} \text{A} \quad \text{A}_1 \quad \text{B} \\ | \quad\;\; | \quad\; | \\ R_3\text{-Lys-Lys-X-(Val)}_n\text{-}R_4 \end{array}$$

worin X Glu oder Asp, $R_3$ Wasserstoff, p-Glu oder eine $\alpha$-Aminoschutzgruppe, n Null oder 1, $R_4$ $NH_2$, einen Benzhydrylaminpolystyrolharzträger oder, wenn n 1 ist, $R_4$ auch $OR_5$ bedeutet, wobei $R_5$ Wasser-

stoff, eine Carboxylschutzgruppe oder —$CH_2$[Polystyrolharzträger] ist, und A und $A_1$ unabhängig voneinander Wasserstoff oder Schutzgruppen für die $\varepsilon$-Aminogruppe von Lysin darstellen, und B Wasserstoff oder eine Schutzgruppe für die Carboxygruppe von Glutamin- oder Asparaginsäure ist, mit der Maßgabe, daß, wenn $R_4$ Amino oder OH ist, zumindest eines von $R_3$, A, $A_1$ und B eine Schutzgruppe bedeutet, und, wenn gewünscht, Überführen der Vererhaltenen Verbindung der Formel (I) in ein pharmazeutisch annehmbares Salz umfaßt.

8. Verfahren zum Herstellen einer Verbindung der Formel (II), wie in Anspruch 1 beansprucht, welches einen der folgenden Schritte umfaßt.

a) Koppeln der erforderlichen Aminosäuren oder Gruppen von Aminosäuren, geeignet geschützt und/oder aktiviert, unter Festphasensynthesebedingungen an ein Benzhydrylamin- oder hydroxy- oder chlormethyliertes Polystyrolharz und, wenn gewünscht, Entfernen einer oder einiger der Schutzgruppen, wobei eine Verbindung der Formel (II) erhalten wird, worin $R_4$ ein Benzhydrylaminharzträger oder O-$CH_2$-[Polystyrolharzträger] ist, oder

b) Koppeln der erforderlichen geeignet geschützen und/oder aktivierten Aminosäuren oder Gruppen von Aminosäuren, in welchen die C-endständige Vorläufer-Aminosäure, wenn notwendig, in Form eines Amids ist, und, wenn gewünscht, Entfernen einer oder eineiger der Schutzgruppen, wobei eine Verbindung der Formel (II) erhalten wird, worin $R_4$ $NH_2$ oder $OR_5$ ist, wobei $R_5$ Wasserstoff oder eine Schutzgruppe darstellt.

9. Eine Verbindung der Formel (I), wie in Anspruch 1 beansprucht, zur Verwendung als immunmodulierendes Mittel.

10. Pharmazeutische Zusammensetzung, die eine Verbindung der Formel (I), wie in Anspruch 1 definiert, oder ein pharmazeutisch annehmbares Salz hievon und einen pharmazeutisch annehmbaren Träger aufweist.

**Patentansprüche für den Vertragsstaat AT**

1. Verfahren zum Herstellen einer Verbindung der Formel (I)

$$R_1\text{-Lys-Lys-X-}R_2 \qquad (I)$$

oder eines pharmazeutisch annehmbaren Salzes hievon, worin $R_1$ Wasserstoff oder p-Glu ist, X Glu oder Asp bedeutet, $R_2$ $NH_2$, Val-$NH_2$ oder Val-OH darstellt, welches das Entfernen jeglicher Schutzgruppen und des Harzträgers, wenn vorhanden, von einer entsprechenden Verbindung der Formel

$$\begin{array}{l} \quad\;\;\; A \quad\; A_1 \; B \\ \quad\;\;\; | \qquad | \quad | \\ R_3\text{-Lys-Lys-X-(Val)}_n\text{-}R_4 \end{array} \qquad (II)$$

worin n Null oder 1 ist, $R_3$ die Bedeutung $R_1$ hat oder eine $\alpha$-aminoschutzgruppe darstellt; $R_4$ $NH_2$, einen Benzhydrylaminharzträger oder, wenn n 1 ist, $R_4$ auch $OR_5$ darstellt, wobei $R_5$ Wasserstoff, eine Carboxyschutzgruppe oder —$CH_2$[Polystyrolharzträger] bedeutet; A und $A_1$ unabhängig voneinander Wasserstoff oder Schutzgruppen für die $\varepsilon$-Aminogruppe von Lysin sind; und B Wasserstoff oder eine Schutzgruppe für die Carboxygruppe von Glutamin- oder Asparaginsäure bedeutet, mit der Maßgabe, daß, wenn $R_4$ Amino oder OH ist, zumindest eines von $R_3$, A, $A_1$ und B eine Schutgruppe ist, umfaßt.

2. Verfahren wie in Anspruch 1 beansprucht, worin $R_3$ eine $\alpha$-Aminoschutzgruppe ist ausgewählt unter Formyl, Trifluoracetyl, Phthalyl, Tosyl, o-Nitrophenylsulfenyl, Benzyloxycarbonyl, p-Nitrobenzyloxycarbonyl, p-Bromobenzyloxycarbonyl, p-Methoxybenzyloxycarbonyl, tert.Butyloxycarbonyl, Diisopropylmethoxycarbonyl, Isopropyloxycarbonyl, Allyloxycarbonyl, 2,2,3-Trichloräthoxycarbonyl, Amyloxycarbonyl, Cyclopentyloxycarbonyl, Adamantyloxycarbonyl, Cyclohexyloxycarbonyl, Phenylthiocarbonyl, Triphenylmethyl und Trimethylsilyl.

3. Verfahren wie in Anspruch 1 oder Anspruch 2 beansprucht, wobei A und $A_1$ jeweils ausgewählt sind unter Tosyl, Amyloxycarbonyl, tert.Butyloxycarbonyl, Diisopropylmethoxycarbonyl, Benzyloxycarbonyl, Halogenbenzyloxycarbonyl und p-Nitrobenzyloxycarbonyl.

4. Verfahren wie in einem der Ansprüche 1 bis 3 beansprucht, wobei B Benzyl ist.

5. Verfahren wie in Anspruch 1 beansprucht, wobei die hergestellte Verbindung der Formel (I) eine der folgenden ist:

p-Glu-Lys-Lys-Glu-Val-$NH_2$
p-Glu-Lys-Lys-Glu-$NH_2$
H-Lys-Lys-Glu-Val-$NH_2$
H-Lys-Lys-Asp-Val-$NH_2$
H-Lys-Lys-Glu-Val-OH oder

ein pharmazeutisch annehmbares Salz hievon.

6. Verfahren zum Herstellen einer Verbindung der Formel

$$\begin{array}{c} \text{A}\quad \text{A}_1\ \text{B} \\ |\quad\ |\quad | \\ \text{R}_3\text{-Lys-Lys-X-(Val)}_n\text{-R}_4 \end{array}$$

oder eines Salzes hievon, worin n Null oder 1 ist, $R_3$ Wasserstoff, p-Glu oder eine $\alpha$-Aminoschutzgruppe bedeutet; X Glu oder Asp darstellt, $R_4$ $NH_2$, einen Benzhydrylaminpolystyrolharzträger oder, wenn n 1 ist, $R_4$ auch $OR_5$ bedeutet, wobei $R_5$ Wasserstoff, eine Carboxyschutzgruppe oder —$CH_2$[Polystyrolharzträger] ist; A und $A_1$ jeweils unabhängig voneinander Wasserstoff oder Schutzgruppen für die $\varepsilon$-Aminogruppe von Lysin sind; und B Wasserstoff oder eine Schutzgruppe für die Carboxygruppe von Glutamin- oder Asparaginsäure bedeutet, mit den Maßgaben, daß (i) wenn $R_4$ Amino oder OH ist, zumindest eines von $R_3$, A, $A_1$ und B eine Schutzgruppe ist, und (ii) wenn $R_3$, A, $A_1$ und B Schutzgruppen sind, n 1 ist und X Glu bedeutet, dann $R_4$ $NH_2$ oder eine Benzhydrylaminharzträger ist, welches umfaßt:

a) Koppeln der erforderlichen Aminosäuren oder Gruppen von Aminosäuren, geeignet geschützt und/oder aktiviert, unter Festphasensynthesebedingungen an einen Benzhydrylamin- oder hydroxy- oder chlormethylierten Polystyrolharzträger und, wenn gewünscht, Entfernen einer oder einiger der Schutzgruppen, wobei ein Verbindung der Formel (II) erhalten wird, worin $R_4$ ein Benzhydrylaminharzträger oder O-$CH_2$-[Polystyrolharzträger] ist, oder

b) Koppeln der erforderlichen geeignet geschützen und/oder aktivierten Aminosäure oder Gruppen von Aminosäuren, in welchen die C-endständige Vorläufer-Aminosäure, wenn notwendig, in Form eines Amids ist, und, wenn gewünscht, Entfernen einer oder einiger der Schutzgruppen, wobei eine Verbindung der Formel (II) erhalten wird, worin $R_4$ $NH_2$ oder $OR_5$ ist, wobei $R_5$ Wasserstoff oder eine Carboxyschutzgruppe ist.

7. Verfahren wie in Anspruch 6 beansprucht, wobei $R_3$, A, $A_1$ und B wie in einem der Ansprüche 2, 3 und 4 definiert sind.

8. Verfahren wie in Anspruch 6 beansprucht, wobei die hergestellte Verbindung der Formel (II) eine der folgenden ist:

L - (5 - Oxoprolyl) - $N^\varepsilon$ - 2 - chlorbenzyloxycarbonyl - L - lysyl - $N^\varepsilon$ - 2 - chlorbenzyloxycarbonyl - L - lysyl - O - benzyl - L - glutamyl - L - valyl - benzhydrylamin - polystyrolamid,

L - (5 - Oxoprolyl) - $N^\varepsilon$ - 2 - chlorbenzyloxycarbonyl - L - lysyl - $N^\varepsilon$ - 2 - chlorbenzyloxycarbonyl - L - lysyl - O - benzyl - L - glutamyl - benzhydrylamin - polystyrolamid,

$N^\alpha$ - t - Boc - $N^\varepsilon$ - 2 - chlorbenzyloxycarbonyl - L - lysyl - $N^\varepsilon$ - 2 - chlorbenzyloxycarbonyl - L - lysyl - O - benzyl - L - glutamyl - L - valyl - benzhydrylamin - polystyrolamid oder

$N^\alpha$ - t - Boc - $N^\varepsilon$ - 2 - chlorbenzyloxycarbonyl - L - lysyl - $N^\varepsilon$ - 2 - chlorbenzyloxycarbonyl - L - lysyl - O - benzyl - L - aspartyl - L - valyl - benzhydrylamin - polystyrolamid.

9. Verfahren zum Herstellen einer pharmazeutischen Zusammensetzung, dadurch gekennzeichnet, daß ein Peptid der Formel (I), wie in Anspruch 1 definiert, oder ein pharmazeutisch annehmbares Salz hievon mit einem pharmazeutischen Träger gemischt und in eine Form gebracht wird, die für therapeutische Verabreichung geeignet ist.

**Revendications pour les Etats Contractants: BE, CH, DE, FR, IT, LI, LU, NL, SE**

1. Polypeptide de formule:

$$R_1\text{-Lys-Lys-X-}R_2 \qquad (I)$$

ou

$$\begin{array}{c} \text{A}\quad \text{A}_1\ \text{B} \\ |\quad\ |\quad | \\ \text{R}_3\text{-Lys-Lys-X-(Val)}_n\text{-R}_4 \end{array} \qquad (II)$$

et ses sels pharmaceutiquement acceptables,

formule dans laquelle X représente Glu ou Asp;

$R_1$ est l'hydrogène ou p-Glu;

$R_2$ est $NH_2$, Val-$NH_2$ ou Val-OH;

$R_3$ représente $R_1$ ou un groupe protégeant la fonction $\alpha$-amino;

*n* est égal à 0 ou à 1;

$R_4$ représente $NH_2$, un support en résine polystyrène benzhydrylaminée ou bien lorsque *n* est égal à 1, $R_4$ représente aussi un groupe $OR_5$ dans lequel $R_5$ est l'hydrogène, un groupe protégeant la fonction carboxyle ou —CH[support de résine polystyrène];

A et $A_1$ représentent, indépendamment, l'hydrogène ou des groupes protecteurs pour le groupe $\varepsilon$-amino de la lysine;

et B est l'hydrogène ou une groupe protecteur pour le groupe carboxy de l'acide glutamique ou aspartique, sous réserve que (i) lorsque $R_4$ est un groupe amino ou OH, l'un au moins de $R_3$, A, $A_1$ et B représente un groupe protecteur et (ii) lorsque $R_3$, A, $A_1$ et B sont des groupes protecteurs, *n* est égal à 1 et X représente Glu, $R_4$ soit un groupe $NH_2$ ou un support de résine benzhydrylaminée.

2. Composé de formule I suivant la revendication 1, qui est l'un des composés suivants:

p-Glu-Lys-Lys-Glu-Val-$NH_2$
p-Glu-Lys-Lys-Glu-$NH_2$
H-Lys-Lys-Glu-Val-$NH_2$
H-Lys-Lys-Glu-Val-OH
H-Lys-Lys-Asp-Val-$NH_2$

ou un sel pharmaceutiquement acceptable de ce composé.

3. Composé de formule II suivant la revendication 1, dans lequel $R_3$ est un groupe protégeant la fonction $\alpha$-amino choisi entre les groupes formyle, trifluoracétyle, phtalyle, tosyle, o-nitrophénylsulfényle, benzyloxycarbonyle, p-nitrobenzyloxycarbonyle, p-bromobenzyloxycarbonyl, p-méthoxybenzyloxycarbonyle, tertio-butyloxycarbonyle, diisopropylméthoxycarbonyl,e isopropyloxycarbonyle, alkoxycarbonyle, 2,2,3-trichloroéthoxycarbonyle, amylcarbonyle, cyclopentyloxycarbonyle adamantyloxycarbonyle, cyclohexyloxycarbonyle, phénylthiocarbonyle, triphénylméthyle et triméthylsilyle.

4. Composé de formule II suivant la revendication 1 ou la revendication 3, dans lequel A et $A_1$ sont choisis chacun entre les groupes tosyle, amyloxycarbonyle, tertio-butyloxycarbonyle, diisopropylméthoxycarbonyle, benzyloxycarbonyle, halogénobenzyloxycarbonyle et p-nitrobenzyloxycarbonyle.

5. Composé de formule II suivant l'une quelconque des revendications 1, 3 et 4, dans lequel B est le groupe benzyle.

6. Composé de formule II suivant la revendication 1, qui est l'un des composés suivants:

L - (5 - oxoprolyl) - $N^\varepsilon$ - 2 - chlorobenzyloxycarbonyl - L - lysyl - $N^\varepsilon$ - 2 - chlorobenzyloxycarbonyl - L - lysyl - O - benzyl - L - glutamyl - L - valylbenzhydrylamine polystyrène amide,

L - (5 - oxoprolyl) - $N^\varepsilon$ - 2 - chlorobenzyloxycarbonyl - L - lysyl - $N^\varepsilon$ - 2 - chlorobenzyloxycarbonyl - L - lysyl - O - benzyl - L - glutamyl - benzhydrylamine polystyrène amide,

$N^\alpha$ - t - Boc - $N^\varepsilon$ - 2 - chlorobenzyloxycarbonyl - L - lysyl - $N^\varepsilon$ - 2 - chlorobenzyloxycarbonyl - L - lysyl - O - benzyl - L - glutamyl - L - valylbenzhydrylamine polystyrène amid ou

$N^\alpha$ - t - Boc - $N^\varepsilon$ - 2 - chlorobenzyloxycarbonyl - *L* - lysyl - $N^\varepsilon$ - 2 - chlorobenzyloxycarbonyl - *L* - lysyl - O - benzyl - L - aspartyl - L - valylbenzhydrylamine polystyrène amide.

7. Procédé de préparation d'un composé de formule I tel que défini dans la revendication 1 ou d'un sel pharmaceutiquement acceptable de ce composé, qui consiste à enlever tous groupes protecteurs et à cliver le support de résine, lorsqu'il est présent, d'un composé de formule II

$$\begin{array}{c} \text{A} \quad \text{A}_1 \quad \text{B} \\ | \qquad | \qquad | \\ \text{R}_3\text{-Lys-Lys-X-(Val)}_n\text{-R}_4 \end{array} \qquad \text{(II)}$$

dans laquelle X représente Glu ou Asp, $R_3$ représente l'hydrogène, p-Glu ou un groupe protégeant la fonction $\alpha$-amino, *n* est égal à 0 ou à 1, $R_4$ représente $NH_2$, un support de résine polystyrène benzhydrylaminée ou bien lorsque *n* est égal à 1, $R_4$ représente également un groupe $OR_5$ dans lequel $R_5$ est l'hydrogène, un groupe protégeant la fonction carboxyle ou un groupe $-CH_2$[support de résine polystyrène], et A et $A_1$ représentant, indépendamment, l'hydrogène ou des groupes protecteurs pour le groupe $\varepsilon$-amino de la lysine; et B est l'hydrogène ou un groupe protecteur pour le groupe carboxy de l'acide glutamique ou aspartique, sous réserve que lorsque $R_4$ est un groupe amino ou OH,l'un au moins de $R_3$, A, $A_1$ et B soit un groupe protecteur, et, le cas échéant, à convertir le composé de formule I obtenu en un sel pharmaceutiquement acceptable.

8. Procédé de préparation d'un composé de formule II suivant la revendication 1, qui comprend l'une des opérations suivantes:

a) couplage des amino-acides ou groupes d'aminoacides convenablement protégés et/ou activés désirés, dans des conditions de synthèse en phase solide, à une résine polystyrène benzhydrylaminée ou hydroxy- ou chloro-méthylée et, le cas échéant, élimination d'un ou plusieurs des groupes protecteurs pour former un composé de formule II dans laquelle $R_4$ est un support de résine benzhydrylaminée ou est du type O-$CH_2$-[support de résine polystyrène], ou

b) couplage des amino-acides ou groupes d'aminoacides convenablement protégés et/ou activés désirés, dans lesquels l'amino-acide C-terminal précurseur est sous la forme d'un amide lorsque cela est nécessaire, le cas échéant, élimination d'un ou plusieurs des groupes protecteurs pour former un composé de formule II dans laquelle $R_4$ est un groupe $NH_2$ ou $OR_5$ où $R_5$ est l'hydrogène ou un groupe protecteur.

9. Composé de formule I suivant la revendication 1, destiné à être utilisé comme agent immunomodulateur.

10. Composition pharmaceutique comprenant un composé de formule I suivant la revendication 1 ou un sel pharmaceutiquement acceptable de ce composé et un support pharmaceutiquement acceptable.

**Revendications pour AT:**

1. Procédé de préparation d'un composé de formule I

$$R_1\text{-Lys-Lys-X-}R_2 \qquad (I)$$

ou d'un sel pharmaceutiquement acceptable de ce composé, formule dans laquelle $R_1$ représente l'hydrogène ou un groupe p-Glu, X est un groupe Glu ou Asp, $R_2$ est un groupe $NH_2$, Val-$NH_2$ ou Val-OH, qui consiste à éliminer tous groupes protecteurs et le support de résine lorsqu'il est présent, d'un composé correspondant de formule:

```
       A    A1  B
       |    |   |
R3-Lys-Lys-X-(Val)n-R4        (II)
```

dans laquelle $n$ est égal à 0 ou à 1, $R_3$ représente $R_1$ ou un groupe protégeant la fonction $\alpha$-amino;

$R_4$ représente $NH_2$, un support de résine benzhydrylaminée ou bien lorsque $n$ est égal à 1, $R_4$ représente également un groupe $OR_5$ dans lequel $R_5$ est l'hydrogène, un groupe protégeant la fonction carboxy ou un groupe —$CH_2$[support de résine polystyrène];

A et $A_1$ représentent, indépendamment, l'hydrogène ou des groupes protégeant le groupe $\varepsilon$-amino de la lysine;

et B est l'hydrogène ou un groupe protéagent le groupe carboxy de l'acide glutamique ou aspartique, sous réserve que lorsque $R_4$ est un groupe amino ou un groupe OH, l'un au moins de $R_3$, A, $A_1$ et B représente un groupe protecteur.

2. Procédé suivant la revendication 1, dans lequel $R_3$ est un groupe protéagent la fonction amino choisi entre les groupes formyle, trifluoroacétyle, phtalyle, tosyle, o-nitrophénylsulfényle, benzyloxycarbonyle, p-nitrobenzyloxycarbonyle, p-bromobenzyloxycarbonyle, p-méthoxybenzyloxycarbonyle, tertio-butyloxycarbonyle, diisopropylméthoxycarbonyle, isopropyloxycarbonyle, allyloxycarbonyle, 2,2,3-trichloréthoxycarbonyle, amyloxycarbonyle, cyclopentyloxycarbonyle, adamantyloxycarbonyle, cyclohexyloxycarbonyle, phénylthiocarbonyle, triphénylméthyle et triméthylsilyle.

3. Procédé suivant la revendication 1 ou la revendication 2, dans lequel A et $A_1$ sont choisis chacun entre les groupes tosyle, amyloxycarbonyle, tertio-butyloxycarbonyle, diisopropylméthoxycarbonyle, benzyloxycarbonyle, halogénobenzyloxycarbonyle et p-nitrobenzyloxycarbonyle.

4. Procédé suivant l'une quelconque des revendications 1 à 3, dans lequel B est le groupe benzyle.

5. Procédé suivant la revendication 1, dans lequel le composé préparé répondant à la formule I est l'un des composés suivants:

p-Glu-Lys-Lys-Glu-Val-$NH_2$
p-Glu-Lys-Lys-Glu-$NH_2$
H-Lys-Lys-Gly-Val-$NH_2$
H-Lys-Lys-Asp-Val-$NH_2$
H-Lys-Lys-Glu-Val-OH

ou un sel pharmaceutiquement acceptable de ce composé.

6. Procédé de préparation d'un composé de formule

```
       A    A1  B
       |    |   |
R3-Lys-Lys-X-(Val)n-R4
```

ou d'un sel de ce composé, formule dans laquelle $n$ est égal à 0 ou à 1, $R_3$ est l'hydrogène, le groupe p-Glu ou un groupe protéageant la fonction $\alpha$-amino; X est une groupe Glu ou Asp, $R_4$ est le group —$NH_2$ ou un support en résine polystyrène benzhydrylaminée ou bien lorsque $n$ est égal à 1, $R_4$ représente également un groupe $OR_5$ dans lequel $R_5$ est l'hydrogène, un groupe protégeant la fonction carboxy ou un groupe —$CH_2$[support de résine polystyrène]; A et $A_1$ représentent chacun, indépendamment, l'hydrogène ou des groupes protecteurs pour le groupe $\varepsilon$-amino de la lysine; et B est l'hydrogène ou un groupe protecteur pour le groupe carboxy de l'acide glutamique ou aspartique, sous réserve que (i) lorsque $R_4$ est un groupe amino ou OH, l'un au moins de $R_3$, A, $A_1$ et B soit un groupe protecteur et (ii)

lorsque $R_3$, A, $A_1$ et B sont des groupes protecteurs, *n* est égal à 1 et X représente Glu, $R_4$ soit un groupe $NH_2$ ou un support de résine benzhydrylaminée, procédé qui consiste

a) à coupler les amino-acides ou groupes d'aminoacides désirés convenablement protégés et/ou activés, dans des conditions de synthèse en phase solide, à une support en résine polystyrène benzhydrylaminée ou hydroxy- ou chlorométhylée et, le cas échéant, à éliminer un ou certains des groupes protecteurs pour obtenir un composé de formule II dans laquelle $R_4$ est un support de résine benzhydrylaminée ou du type $OCH_2$[support de résine polystyrène], ou

b) à coupler l'amino-acide ou les groupes d'aminoacides convenablement protégés et/ou activés désirés, dont l'amino-acide à précurseur C-terminal est sous la forme d'un amide lorsque cela est nécessaire, pour obtenir un composé de formule II dans laquelle $R_4$ représente $NH_2$ ou un groupe $OR_5$ dans lequel $R_5$ est l'hydrogène ou un groupe protéagent la fonction carboxy.

7. Procédé suivant la revendication 6, dans lequel $R_3$, A, $A_1$ et B ont les définitions données dans l'une quelconque des revendications 2, 3 et 4.

8. Procédé suivant la revendication 6, dans lequel le composé de formule II préparé est l'un des composés suivants:

L - (5 - oxoprolyl) - $N^{\varepsilon}$ - 2 - chlorobenzyloxycarbonyl - L - lysyl - $N^{\varepsilon}$ - 2 - chlorobenzyloxycarbonyl - L - lysyl - O - benzyl - L - glutamyl - L - valylbenzhydrylamine polystyrène amide,

L - (5 - oxoprolyl) - $N^{\varepsilon}$ - 2 - chlorobenzyloxycarbonyl - L - lysyl - $N^{\varepsilon}$ - 2 - chlorobenzyloxycarbonyl - L - lysyl - O - benzyl - L - glutamylbenzhydrylamine polystyrène amide,

$N^{\alpha}$ - t - Boc - $N^{\varepsilon}$ - 2 - chlorobenzyloxycarbonyl - L - lysyl - $N^{\varepsilon}$ - 2 - chlorobenzyloxycarbonyl - L - lysyl - O - benzyl - L - glutamyl - L - valylbenzhydrylamine polystyrène amide, ou

$N^{\alpha}$ - t - Boc - $N^{\varepsilon}$ - 2 - chlorobenzyloxycarbonyl - *L* - lysyl - $N^{\varepsilon}$ - 2 - chlorobenzyloxycarbonyl - *L* - lysyl - O - benzyl - *L* - aspartyl - *L* - valyl - benzhydrylamine polystyrène amide.

9. Procédé de préparation d'une composition pharmaceutique, caractérisé en ce qu'un peptide de formule I tel que défini dans la revendication 1 ou un sel pharmaceutiquement acceptable de ce composé est mélangé avec un support pharmaceutique et le mélange est mis sous une forme propre à l'administration thérapeutique.